## Europäisches Patentamt
## European Patent Office
## Office européen des brevets

(19)

(11) Veröffentlichungsnummer: **0 009 715**
**B1**

(12) # EUROPÄISCHE PATENTSCHRIFT

(45) Veröffentlichungstag der Patentschrift:
**16.06.82**

(51) Int. Cl.³: **C 07 G 7/00**, G 01 N 33/48

(21) Anmeldenummer: **79103517.3**

(22) Anmeldetag: **19.09.79**

(54) **Neues ubiquitäres Gewebsprotein PP8 und Verfahren zu seiner Anreicherung.**

(30) Priorität: **29.09.78 DE 2842467**

(43) Veröffentlichungstag der Anmeldung:
**16.04.80 Patentblatt 80/8**

(45) Bekanntmachung des Hinweises auf die Patenterteilung:
**16.06.82 Patentblatt 82/24**

(84) Benannte Vertragsstaaten:
**AT BE CH DE FR GB IT LU NL SE**

(56) Entgegenhaltungen:
**DE-A1-2 445 677**
**DE-A1-2 640 387**
**DE-A1-2 720 704**
**DE-A1-2 726 886**

(73) Patentinhaber: **BEHRINGWERKE AKTIENGESELLSCHAFT, Postfach 1140, D-3550 Marburg/Lahn (DE)**

(72) Erfinder: **Bohn, Hans, Dr., Oberer Eichweg 26, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Neues ubiquitäres Gewebsprotein PP$_8$ und Verfahren zu seiner Anreicherung

Die Erfindung betrifft ein neues, lösliches Gewebsprotein (PP$_8$) und ein Verfahren zu dessen Anreicherung aus Organgeweben.

In der deutschen Offenlegungsschrift 2 640 387 ist ein gewebespezifisches Protein beschrieben, das nicht mit dem erfindungsgemässen Protein identisch ist.

PP$_8$ kommt in fast allen bisher untersuchten Organen, vor allem in der Plazenta des Menschen vor. Es wurde in folgenden fetalen Organen nachgewiesen: Herz, Leber, Niere, Lunge, Magen, Gehirn, ferner in folgenden adulten Organen: Herz, Lunge, Magen, Niere, Uterus, Leber, Milz, Nebenniere, Colon und Blase. Aus einer menschlichen ausgewachsenen Plazenta (600 g) lassen sich mit physiologischer Salzlösung im Durchschnitt etwa 10 mg dieses Proteins extrahieren. In ähnlicher Grössenordnung dürfte die Konzentration von PP$_8$ in anderen menschlichen Organen sein. Im Serum kommt PP$_8$ normalerweise nicht oder nur in Spuren ($< 0,1$ mg pro 100 ml) vor.

Gegenstand der Erfindung ist das Gewebsprotein PP$_8$, gekennzeichnet durch

a) einen Proteinanteil von $96 \pm 3\%$, einen Gehalt an Kohlenhydraten von $4,1 \pm 0,95\%$ und davon Hexosen $3,15 \pm 0,5\%$, Hexosamin $0,61 \pm 0,2\%$, Fucose $0,11 \pm 0,05$, Neuraminsäure $0,23 \pm 0,20\%$;

b) einen Sedimentationskoeffizienten S$_{20,w}$ von $3,7 \pm 0,3$ S;

c) ein in der Ultrazentrifuge bestimmtes Molekulargewicht von $45\,000 \pm 5000$;

d) ein im Natriumdodecylsulfat (SDS)-haltigen Polyacrylamidgel bestimmtes Molekulargewicht von $55\,000 \pm 5000$;

e) einen Extinktionskoeffizienten E$^{1\%}_{1\,cm}$ (280 nm) von $8,1 \pm 1,0$;

f) eine elektrophoretische Beweglichkeit im Bereich der alpha$_1$-Globuline;

g) einen isoelektrischen Punkt von $4,7 \pm 0,3$.

h) eine Aminosäurenzusammensetzung:

| | Mol-% | Variations-koeffizient (%) |
|---|---|---|
| Lysin | 7,08 | 3,21 |
| Histidin | 1,49 | 22,07 |
| Arginin | 3,56 | 3,86 |
| Asparaginsäure | 10,32 | 6,79 |
| Thronin | 6,08 | 0,96 |
| Serin | 7,88 | 3,99 |
| Glutaminsäure | 12,97 | 3,48 |
| Prolin | 2,87 | 4,84 |
| Glycin | 6,64 | 2,19 |
| Alanin | 6,41 | 0,65 |
| Cystin/2 | 2,00 | 17,75 |
| Valin | 5,91 | 3,81 |
| Methionin | 4,67 | 14,45 |
| Isoleucin | 3,10 | 2,01 |
| Leucin | 9,29 | 3,58 |
| Tyrosin | 1,65 | 14,40 |
| Phenylalanin | 6,67 | 7,43 |
| Tryptophan | 1,41 | 2,13 |

und

i) die Fähigkeit, die Bildung spezifischer Antikörper auszulösen.

Zur Erläuterung der kennzeichnenden Merkmale des Gewebsproteins sei folgendes ausgeführt:

Die Bestimmung der Sedimentationskoeffizienten wurde in einer analytischen Ultrazentrifuge der Firma Beckman (Spinco-Apparatur, Modell E) bei 66 000 UpM in Doppelsektorzellen mit Hilfe der UV-Scanner Technik bei 280 nm durchgeführt. Als Lösungsmittel diente ein 0,05 M Phosphatpuffer (pH 6,8), der 0,2 Mol/l NaCl enthielt. Die Proteinkonzentration wurde auf eine optische Dichte von etwa 3 eingestellt. Die Sedimentationskoeffizienten sind auf die Basis von Wasser bei 20 °C umgerechnet worden.

Zur Ermittlung des Molekulargewichts in der UZ wurde die Sedimentationsgleichgewichtsmethode herangezogen. Die Konzentration ist dabei auf ca. 1.0 O.D. eingestellt worden. Die Bestimmung wurde bei 9000 UpM vorgenommen. Die Registrierung erfolgte mit UV-Optik bei 280 nm unter Einsatz eines photoelektrischen Scanners.

Zur Bestimmung des Molekulargewichts im SDS-PAA-Gel wurde ein Gel mit 7,5% Polyacrylamid (PAA), das 0,1% Natriumdodecylsulfat (SDS) enthielt, verwendet. Als Vergleichssubstanz dienten humanes Plazentalaktogen (HPL) und Human-Albumin sowie dessen Aggregate.

Zur Bestimmung des Extinktionskoeffizienten wurde die Substanz 0,10%ig in Aqua dest. gelöst.

Die Untersuchung der elektrophoretischen Beweglichkeit erfolgte auf Zelluloseazetatfolien der Firma Satorius mit Natriumdiäthylbarbiturat-Puffer pH 8,6 und unter Verwendung des Gerätes Microzone R 200 der Firma Beckman Instruments.

Die Bestimmung des isoelektrischen Punktes wurde mit einer Säule (440 ml) der Firma LKB, Stockholm, durchgeführt. Das sogenannte Ampholin®-Gemisch hatte bei der Untersuchung des Glykoproteins einen pH-Bereich von 4,0 bis 6,0.

Die Bestimmung der Kohlenhydrate erfolgte nach der von H.E. Schultze, R. Schmidtberger, H. Haupt, Biochem. Z., 329, Seite 490 (1958), beschriebenen Methode.

Die Aminosäurenanalyse wurde nach S. Moore, D.H. Spackmann, W.H. Stein, Anal. Chem. 30, S. 1185 (1958), unter Verwendung des Flüssigkeitschromatographen Multichrom B der Firma Beckman durchgeführt. ½Cystin wurde nach Oxydation der Proteine mit Perameisensäure [S. Moore et al., Anal. Chem. 30, S. 1185 (1958)] und nachfolgender Chromatographie [S. Moore, J. Biol. Chem., 238, S. 235 (1963)] als Cysteinsäure bestimmt. Der Tryptophangehalt ist mit der direkten photometrischen Bestimmung nach H. Edelhoch, Biochemistry, 6, S. 1948 (1967), ermittelt worden.

Die Ergebnisse der Aminosäurenanalyse des nach dem Beispiel erhältlichen PP$_8$ sind in Tabelle I zusammengestellt.

Tabelle I
Aminosäurenzusammensetzung von $PP_8$

|  | (Mol-%) | Variations-koeffizient (VK) (%) |
|---|---|---|
| Lysin | 7,08 | 3,21 |
| Histidin | 1,49 | 22,07 |
| Arginin | 3,56 | 3,86 |
| Asparaginsäure | 10,32 | 6,79 |
| Threonin | 6,08 | 0,96 |
| Serin | 7,88 | 3,99 |
| Glutaminsäure | 12,97 | 3,48 |
| Prolin | 2,87 | 4,84 |
| Glycin | 6,64 | 2,19 |
| Alanin | 6,41 | 0,65 |
| Cystin/2 | 2,00 | 17,75 |
| Valin | 5,91 | 3,81 |
| Methionin | 4,67 | 14,45 |
| Isoleucin | 3,10 | 2,01 |
| Leucin | 9,29 | 3,58 |
| Tyrosin | 1,65 | 14,40 |
| Phenylalanin | 6,67 | 7,43 |
| Tryptophan | 1,41 | 2,13 |

Für $PP_8$ wurden folgende Eigenschaften festgestellt, die sich zur Isolierung des neuen Gewebsproteins verwenden lassen:

1) Mit Ammoniumsulfat wird $PP_8$ bei pH 7,0 zwischen 40–70% Sättigung aus wässrigen Lösungen gefällt.

2) Mit wasserlöslichen Akridinbasen, z.B. 2-Äthoxi-6,9-diaminoacridinlactat (Rivanol®) wird $PP_8$ bei pH-Werten zwischen 7–9 und einer Konzentration von 0,4 bis 0,8 g/100 ml präzipitiert.

3) Bei der präparativen Elektrophorese wandert $PP_8$ im Bereich der $\alpha_2$-Globuline.

4) Bei der Gelfiltration (Sephadex®) erscheint $PP_8$ im Bereich der Proteine mit Molekulargewichten von 30 000 bis 70 000.

5) $PP_8$ lässt sich an schwach basische Ionenaustauscher wie z.B. DEAE-Zellulose oder DEAE-Sephadex® bei niedriger Leitfähigkeit (etwa 0–2 mS) und neutralem oder schwach alkalischem pH-Wert (etwa pH 7 bis 9) adsorbieren.

6) Beim Erwärmen (4 Stunden 67 °C bei pH 5,0) in Gegenwart von Salzen aliphatischer Fettsäuren wie Caprylat bleibt $PP_8$, ähnlich wie dies von Albumin bekannt ist, in Lösung, während die meisten anderen Proteine denaturieren und ausfallen.

7) $PP_8$ kann aus seiner wässrigen Lösung durch Immunadsorption angereichert und isoliert werden.

Gegenstand der Erfindung sind ferner Verfahren zur Herstellung des $PP_8$, dadurch gekennzeichnet, dass wässrige Organextrakte, vor allem ein entsprechender Extrakt aus der Plazenta, aber auch andere wässrige Lösungen, die dieses Protein enthalten, unter Zugrundelegung der obenstehenden Eigenschaften fraktioniert werden. Neben Ammoniumsulfat ist selbstverständlich, dass auch andere in der präparativen Biochemie üblicherweise eingesetzte Neutralsalze zur Ausfällung des $PP_8$ verwendet werden können. Neben Akridinbasen sind auch wasserlösliche Derivate einer Chinolinbase, die bei Proteinfraktionierungen Verwendung finden, im Rahmen des erfindungsgemässen Verfahrens einsetzbar. Entsprechend seinen elektrophoretischen Verhalten wie dem erfindungsgemäss festgestellten Molekulargewicht können zur Isolierung des Proteins auch andere Massnahmen angewandt werden, die geeignet sind, ein $\alpha_1$-Globulin von übrigen Plasma- oder Gewebeproteinen abzutrennen. Im Hinblick auf das Molekulargewicht könnten hierzu die verschiedenen Methoden der Gelfiltration, Gelchromatographie oder Ultrafiltration eingesetzt werden. Dies zeigt sich auch in der Eigenschaft des $PP_8$, an schwach basische Ionenaustauscher gebunden und hiervon wieder eluiert werden zu können.

Durch eine ausgewählte Kombination der genannten Massnahmen, die einerseits zur Anreicherung des $PP_8$, anderseits zur Abtrennung dieses Proteins von den übrigen Gewebeproteinen bzw. Plasmaproteinen führen, kann die Isolierung der erfindungsgemäss gefundenen Substanz erfolgen. Demzufolge ist der Gegenstand der vorliegenden Erfindung in den einzelnen Anreicherungsschritten für das $PP_8$ und in den durch Kombination der Massnahmen zur Anreicherung sich ergebenden Verfahren zur Reinigung des $PP_8$ zu sehen.

Das Verfahren zur Anreicherung ist gekennzeichnet durch die Anwendung mindestens einer der Massnahmen 1 bis 7.

$PP_8$ hat antigene Eigenschaften; bei der Immunisierung von Tieren mit diesem Protein werden spezifische Antikörper gebildet. Der Nachweis und die Bestimmung des $PP_8$ mit immunologischen Methoden hat diagnostische Bedeutung. $PP_8$ ist ein offensichtlich in fast allen menschlichen Organen vorkommendes Gewebsprotein. Bei Erkrankungen, die mit Gewebszerfall einhergehen, ist dieses Protein in erhöhter Konzentration im Blut nachweisbar. Seine Bestimmung kann daher ganz allgemein zur Erkennung von Erkrankungen sowie zur Überwachung des Krankheitsverlaufs und zur Kontrolle der Therapie verwendet werden.

Die Erfindung wird am nachstehenden Beispiel näher erläutert:

Beispiel:

A) Extraktion der Plazenten und Fraktionierung des Extraktes

150 g tiefgefrorene menschliche Plazenten werden im Schneidmischer zerkleinert und mit 150 l einer 0,4%igen Kochsalzlösung extrahiert. Der Extrakt wird dann, nach Abtrennung des Geweberückstandes durch Zentrifugation, mit 20%iger Essigsäure auf pH 6,0 eingestellt und unter Rühren mit 33 l einer 3%igen Lösung von 2-Äthoxy-6,9-Diaminoacridin-Lactat (Rivanol-®, Hoechst AG) versetzt. Der entstehende Niederschlag wird abzentrifugiert und verworfen. Den Überstand stellt man mit 2 N Natronlauge auf pH 8,5 ein und gibt unter Rühren nochmals langsam 50 l einer

3%igen Lösung des 2-Äthoxy-6,9-diaminoacridin-Lactats hinzu. PP$_8$ geht dabei zur Hauptsache in die Fällung; der Überstand wird abgehebert und verworfen. Die Fällung wird mit 120 l Wasser verrührt und durch Zugabe von 20%iger Essigsäure bis zu einem pH-Wert von 5,0 gelöst. Nach 2stündigem Rühren setzt man zur Verdrängung der Acridinbase (Rivanol-®) 5 g/100 ml Kochsalz zu und stellt den pH-Wert auf 7,0 ein. Das ausgeschiedene Rivanol-Hydrochlorid wird abfiltriert. Das eiweisshaltige Filtrat versetzt man mit 0,20 kg Natriumkaprylat (gelöst in 15 l Wasser) und stellt dann den pH-Wert der Lösung durch Zugabe von 20%iger Essigsäure auf 5.0 ein. Unter ständigem Rühren wird die Lösung auf 67 °C erhitzt und 4 Stunden bei dieser Temperatur gehalten. Anschliessend kühlt man wieder auf 10–15°C ab. Die meisten Proteine werden bei dieser Hitzebehandlung denaturiert und fallen aus; Albumin und das neue Protein PP$_8$ bleiben in Lösung. Der Niederschlag wird abfiltriert und die Lösung nach Neutralisation auf einem Ultrafilter bis auf ca. 20% Eiweiss ankonzentriert. Ausbeute 3,5 l, enthaltend 5–10 mg PP$_8$ pro 100 ml. Aus dieser Lösung wurde PP$_8$ weiter durch Immunadsorption angereichert und isoliert.

B. Anreicherung von PP$_8$ durch Immunadsorption

1. Herstellung des Immunadsorbens

300 ml eines Anti-PP$_8$-Serums vom Kaninchen werden gegen 0,02 M Phosphatpuffer (pH 7,0) dialysiert und zur Abtrennung der Immunglobuline an DEAE-Zellulose chromatographiert. Die Immunglobulinfraktion (3,75 g Protein) wird dann mit 375 g besonders gereinigter Agarose in Kugelform (Sepharose® 4B der Pharmacia, Uppsala, Schweden), die mit 46,9 g Bromcyan aktiviert worden war, umgesetzt und so kovalent an einen Träger gebunden.

Das Verfahren ist beschrieben von Axen, R., Porath, J., Ernbach, S., Nature, 214, 1302 (1967).

Mit Hilfe eines auf diese Weise hergestellten Immunadsorbens kann das Plazentaprotein PP$_8$ aus dessen Lösung, insbesondere aus PP$_8$-angereicherten Plazentaextraktfraktionen isoliert werden.

2. Durchführung der Immunadsorption

Das Immunadsorbens wird in 0,1 M Tris–HCl–Puffer (pH 8,0), der 1,0 Mol/l NaCl und 0,1%NaN$_3$, (nachstehend Pufferlösung I) enthält, suspendiert, dann in eine Chromatographiesäule gefüllt (5,5 × 20 cm) und mit Pufferlösung I nachgespült. Dann lässt man langsam 450 ml der PP$_8$-haltigen Lösung durch die Säule wandern, wobei PP$_8$ immunadsorptiv gebunden wird. Man wäscht die Säule gründlich mit Puffer I nach und eluiert dann das adsorbierte Protein mit 3 M Kaliumrhodanid-Lösung. Die PP$_8$-haltigen Eluate werden gegen Pufferlösung I dialysiert und im Ultrafilter auf ca. 10 ml eingeengt. Ausbeute pro Adsorption ~10 mg PP$_8$.

Das Adsorbens in der Säule wird unmittelbar nach der Elution von PP$_8$ wieder mit Pufferlösung I neutralisiert und gründlich gewaschen; es kann dann erneut zur (immun)-adsorptiven Bindung von PP$_8$ eingesetzt werden.

C. Hochreinigung von PP$_8$

Das durch Immunadsorption gewonnene Protein ist häufig durch unspezifisch gebundene Serumproteine (hauptsächlich Albumin) verunreinigt. Die Abtennung der Hauptmenge der Begleitproteine gelingt z.B. durch Gelfiltration an Sephadex®G-150. PP$_8$ kommt dabei unmittelbar nach dem Albumin von der Säule. Die restlichen Serumproteine können durch inverse oder negative Immunadsorption, d.h. mit Hilfe von trägergebundenen Antikörpern gegen die als Verunreinigung vorliegenden Serumproteine, entfernt werden.

**Patentansprüche für die Vertragsstaaten: BE, CH, DE, FR, GB, IT, LU, NL, SE**

1. Gewebeprotein PP$_8$, gekennzeichnet durch

a) einen Proteinanteil von 96 ± 3%, einen Gehalt an Kohlenhydraten von 4,1 ± 0,95% und davon Hexosen 3,15 ± 0,5%, Hexosamin 0,61 ± 0,2%, Fucose 0,11 ± 0,05. Neuraminsäure 0,23 ± 0,20%;

b) einen Sedimentationskoeffizienten $S_{20,W}$ von 3,7 ± 0,3 S;

c) ein in der Ultrazentrifuge bestimmtes Molekulargewicht von 45 000 ± 5000;

d) ein im Natriumdodezylsulfat (SDS)-haltigen Polyacrylamidgel bestimmtes Molekulargewicht von 55 000 ± 5000;

e) einen Extinktionskoeffizienten $E^{1\%}_{1\,cm}$ (280 nm) von 8,1 ± 1,0;

f) eine elektrophoretische Beweglichkeit im Bereich der $\alpha_1$-Globuline;

g) einen isoelektrischen Punkt von 4,7 ± 0,3;

h) eine Aminosäurenzusammensetzung:

| | Mol-% | Variations-koeffizient (%) |
|---|---|---|
| Lysin | 7,08 | 3,21 |
| Histidin | 1,49 | 22,07 |
| Arginin | 3,56 | 3,86 |
| Asparaginsäure | 10,32 | 6,79 |
| Threonin | 6,08 | 0,96 |
| Serin | 7,88 | 3,99 |
| Glutaminsäure | 12,97 | 3,48 |
| Prolin | 2,87 | 4,84 |
| Glycin | 6,64 | 2,19 |
| Alanin | 6,41 | 0,65 |
| Cystin/2 | 2,00 | 17,75 |
| Valin | 5,91 | 3,81 |
| Methionin | 4,67 | 14,45 |
| Isoleucin | 3,10 | 2,01 |
| Leucin | 9,29 | 3,58 |
| Tyrosin | 1,65 | 14,40 |
| Phenylalanin | 6,67 | 7,43 |
| Tryptophan | 1,41 | 2,13 |

und

i) die Fähigkeit, die Bildung spezifischer Antikörper auszulösen.

2. Verfahren zur Anreicherung des Gewebe-

proteins PP$_8$ nach Anspruch 1, dadurch gekennzeichnet, dass wässrige Organextrakte oder daraus gewonnene Lösungen, die dieses Protein enthalten, mindestens einer der folgenden Massnahmen unterworfen werden und die bezüglich des Gewebsproteins PP$_8$ angereicherte Fraktion gewonnen wird:

1) Fällung mit Ammoniumsulfat im pH-Bereich von 5 bis 8 zwischen 40–70% Sättigung;

2) Fällung mit einer wasserlöslichen Akridinbase bei einem pH-Wert zwischen 7 und 9 und einer Konzentration von 0,4 bis 0,8 g/100 cm³;

3) präparative Zonenelektrophorese und Gewinnung der alpha$_1$-Globulinfraktion;

4) Gelfiltration zur Gewinnung von Proteinen im Molekulargewichtsbereich von 30 000 bis 70 000;

5) Adsorption an schwach basische Ionenaustauscher und Elution des Gewebsproteins;

6) Erwärmen der wässrigen Lösung in Gegenwart von Salzen aliphatischer Fettsäuren zur Ausfällung von Verunreinigungen des PP$_8$ und

7) immunadsorptive Anreicherung.

**Patentanspruch für den Vertragsstaat: AT**

Verfahren zur Anreicherung des Gewebeproteins PP$_8$ mit

a) einem Proteinanteil von $96 \pm 3\%$, einem Gehalt an Kohlenhydraten von $4,1 \pm 0,95\%$, davon Hexosen $3,15 \pm 0,5\%$, Hexosamin $0,61 \pm 0,2\%$, Fucose $0,11 \pm 0,05$ und Neuraminsäure $0,23 \pm 0,20\%$;

b) einem Sedimentationskoeffizienten $S_{20,W}$ von $3,7 \pm 0,3$ S;

c) einem in der Ultrazentrifuge bestimmten Molekulargewicht von $45\,000 \pm 5000$;

d) einem in Natriumdodecylsulfat (SDS)-haltigem Polyacrylamidgel bestimmten Molekulargewicht von $55\,000 \pm 5000$;

e) einem Extinktionskoeffizienten $E^{1\%}_{1\,cm}$ (280 nm) von $8,1 \pm 1,0$;

f) einer elektrophoretischen Beweglichkeit im Bereich der alpha$_1$-Globuline;

g) einem isoelektrischen Punkt von $4,7 \pm 0,3$;

h) einer Aminosäurenzusammensetzung:

| | Mol-% | Variations-koeffizient (%) |
|---|---|---|
| Lysin | 7,08 | 3,21 |
| Histidin | 1,49 | 22,07 |
| Arginin | 3,56 | 3,86 |
| Asparaginsäure | 10,32 | 6,79 |
| Threonin | 6,08 | 0,96 |
| Serin | 7,88 | 3,99 |
| Glutaminsäure | 12,97 | 3,48 |
| Prolin | 2,87 | 4,84 |
| Glycin | 6,64 | 2,19 |
| Alanin | 6,41 | 0,65 |
| Cystin/2 | 2,00 | 17,75 |
| Valin | 5,91 | 3,81 |
| Methionin | 4,67 | 14,45 |
| Isoleucin | 3,10 | 2,01 |
| Leucin | 9,29 | 3,58 |
| Tyrosin | 1,65 | 14,40 |
| Phenylalanin | 6,67 | 7,43 |
| Tryptophan | 1,41 | 2,13 |

und

i) der Fähigkeit, die Bildung spezifischer Antikörper auszulösen,

dadurch gekennzeichnet, dass wässrige Organextrakte oder daraus gewonnene Lösungen, die dieses Protein enthalten, mindestens einer der folgenden Massnahmen unterworfen werden und die bezüglich des Gewebeproteins PP$_8$ angereicherte Fraktion gewonnen wird:

1) Fällung mit Ammoniumsulfat im pH-Bereich von 5 bis 8 zwischen 40–70% Sättigung;

2) Fällung mit einer wasserlöslichen Akridinbase bei einem pH-Wert zwischen 7 und 9 und einer Konzentration von 0,4 bis 0,8 g/100 cm³;

3) präparative Zonenelektrophorese und Gewinnung der alpha$_1$-Globulinfraktion;

4) Gelfiltration zur Gewinnung von Proteinen im Molekulargewichtsbereich von 30 000 bis 70 000;

5) Adsorption an schwach basische Ionenaustauscher und Elution des Gewebeproteins;

6) Erwärmen der wässrigen Lösung in Gegenwart von Salzen aliphatischer Fettsäuren zur Ausfällung von Verunreinigungen des PP$_8$ und

7) immunadsorptive Anreicherung.

**Claims for the contracting states: BE, CH, DE, FR, GB, IT, LU, NL, SE**

1. Tissue protein PP$_8$, characterized by

a) a protein proportion of $96 \pm 3\%$, a content of carbohydrates of $4.1 \pm 0.95\%$, of which are $3.15 \pm 0.5\%$ hexoses, $0.61 \pm 0.2$ hexosamine, $0.11 \pm 0.05\%$ fucose and $0.23 \pm 0.20$ neuraminic acid;

b) a sedimentation coefficient $S_{20,W}$ of $3.7 \pm 0.3$ S;

c) a molecular weight of $45\,000 \pm 5000$ determined in the ultracentrifuge;

d) a molecular weight of $55\,000 \pm 5000$ determined in sodium dodecylsulfate (SDS)-containing polyacrylamide gel;

e) an extinction coefficient $E^{1\%}_{1\,cm}$ (280 nm) of $8.1 \pm 1.0$;

f) an electrophoretic mobility in the range of the alpha$_1$-globulins;

g) an isoelectric point of $4.7 \pm 0.3$;

h) the amino-acid composition

| Amino acid | Mole % | Variation coefficient (%) |
|---|---|---|
| Lysine | 7.08 | 3.21 |
| Histidine | 1.49 | 22.07 |
| Arginine | 3.56 | 3.86 |
| Aspartic acid | 10.32 | 6.79 |
| Threonine | 6.08 | 0.96 |
| Serine | 7.88 | 3.99 |
| Glutamic acid | 12.97 | 3.48 |
| Proline | 2.87 | 4.84 |
| Glycine | 6.64 | 2.19 |
| Alanine | 6.41 | 0.65 |
| Cystine/2 | 2.00 | 17.75 |
| Valine | 5.91 | 3.81 |
| Methionine | 4.67 | 14.45 |
| Isoleucine | 3.10 | 2.01 |

| Amino acid | Mole % | Variation coefficient (%) |
| --- | --- | --- |
| Leucine | 9.29 | 3.58 |
| Tyrosine | 1.65 | 14.40 |
| Phenylalanine | 6.67 | 7.43 |
| Tryptophane | 1.41 | 2.13 |

and

i) the property to cause the formation of specific anti-bodies.

2. Process for enriching the tissue protein $PP_8$ claimed in claim 1, which comprises subjecting aqueous organ extracts or solutions obtained therefrom, which contain this protein, to at least one of the following measures and isolating the fraction containing the enriched tissue protein $PP_8$:

1) precipitation with ammonium sulfate in the pH-range of from 5 to 8 at a saturation of 40 to 70%;

2) precipitation with a water-soluble acridine base at a pH-value of between 7 and 9 and a concentration of 0.4 to 0.8 g/100 cm³;

3) preparative zone electrophoresis and isolation of the alpha₁-globulin fraction;

4) gel-filtration for the isolation of proteins in the range of the molecular weight of 30 000 to 70 000;

5) adsorption on weakly basic ion exchangers and elution of the tissue protein;

6) heating of the aqueous solution in the presence of salts of aliphatic fatty acids for precipitating contaminations of the $PP_8$ and

7) immuno-adsorptive enrichment.


**Claim for the contracting state: AT**

Process for enriching the tissue protein $PP_8$ with

a) a protein proportion of $96 \pm 3\%$, a content of carbohydrates of $4.1 \pm 0.95\%$, of which are $3.15 \pm 0.5\%$ hexoses, $0.61 \pm 0.2$ hexosamine, $0.11 \pm 0.05\%$ fucose and $0.23 \pm 0.20$ neuraminic acid;

b) a sedimentation coefficient $S_{20,W}$ of $3.7 \pm 0.3$ S;

c) a molecular weight of $45\,000 \pm 5000$ determined in the ultracentrifuge;

d) a molecular weight of $55\,000 \pm 5000$ determined in sodium dodecylsulfate (SDS)-containing polyacrylamide gel;

e) an extinction coefficient $E^{1\%}_{1\,cm}$ (280 nm) of $8.1 \pm 1.0$;

f) an electrophoretic mobility in the range of the alpha₁-globulins;

g) an isoelectric point of $4.7 \pm 0.3$;

h) the amino-acid composition

| Amino acid | Mole % | Variation coefficient (%) |
| --- | --- | --- |
| Lysine | 7.08 | 3.21 |
| Histidine | 1.49 | 22.07 |
| Arginine | 3.56 | 3.86 |
| Aspartic acid | 10.32 | 6.79 |
| Threonine | 6.08 | 0.96 |

| Amino acid | Mole % | Variation coefficient (%) |
| --- | --- | --- |
| Serine | 7.88 | 3.99 |
| Glutamic acid | 12.97 | 3.48 |
| Proline | 2.87 | 4.84 |
| Glycine | 6.64 | 2.19 |
| Alanine | 6.41 | 0.65 |
| Cystine/2 | 2.00 | 17.75 |
| Valine | 5.91 | 3.81 |
| Methoionine | 4.67 | 14.45 |
| Isoleucine | 3.10 | 2.01 |
| Leucine | 9.29 | 3.58 |
| Tyrosine | 1.65 | 14.40 |
| Phenylalanine | 6.67 | 7.43 |
| Tryptophane | 1.41 | 2.13 |

and

i) the property to cause the formation of specific anti-bodies, which comprises subjecting aqueous organ

extracts or solutions obtained therefrom, which contain this protein, to at least one of the following measures and isolating the fraction containing the enriched tissue protein $PP_8$:

1) precipitation with ammonium sulfate in the pH-range of from 5 to 8 at a saturation of 40 to 70%;

2) precipitation with a water-soluble acridine base at a pH-value of between 7 and 9 and a concentration of 0,4 to 0,8 g/100 cm³;

3) preparative zone electrophoresis and isolation of the alpha₁-globulin fraction;

4) gel-filtration for the isolation of proteins in the range of the molecular weight of 30 000 to 70 000;

5) adsorption on weakly basic ion exchangers and elution of the tissue protein;

6) heating of the aqueous solution in the presence of salts of aliphatic fatty acids for precipitating contaminations of the $PP_8$ and

7) immuno-adsorptive enrichment.


**Revendications pour les Etats contractants: BE, CH, DE, FR, GB, IT, LU, NL, SE**

1. Protéine tissulaire $PP_8$, caractérisée par

a) une teneur en protéines de $96 \pm 3\%$, une teneur en hydrates de carbone de $4,1 \pm 0,95\%$, parmi lesquels $3,15 \pm 0,5\%$ d'hexoses, $0,61 \pm 0,2\%$ d'hexosamine, $0,11 \pm 0,05\%$ de fucose, $0,23\% \pm 0,20\%$ d'acide neuraminique;

b) un coefficient de sédimentation $S_{20,W}$ de $3,7 \pm 0,3$ S;

c) une masse moléculaire déterminée par ultracentrifugation de $45\,000 \pm 5000$;

d) une masse moléculaire déterminée dans un gel de polyacrylamide contenant du dodécylsulfate de sodium (SDS) de $55\,000 \pm 5000$;

e) un coefficient d'extinction $E^{1\%}_{1\,cm}$ (280 nm) de $8,1 \pm 1,0$;

f) une mobilité électrophorétique dans la région des α₁-globulines;

g) un point isoélectrique de $4,7 \pm 0,3$;

h) la composition en aminoacides suivante:

|  | Moles % | Coefficent de variation (%) |
|---|---|---|
| Lysine | 7,08 | 3,21 |
| Histidine | 1,49 | 22,07 |
| Arginine | 3,56 | 3,86 |
| Acide aspartique | 10,32 | 6,79 |
| Thréonine | 6,08 | 0,96 |
| Sérine | 7,88 | 3,99 |
| Acide glutamique | 12,97 | 3,48 |
| Proline | 2,87 | 4,84 |
| Glycine | 6,64 | 2,19 |
| Alanine | 6,41 | 0,65 |
| Cystine/2 | 2,00 | 17,75 |
| Valine | 5,91 | 3,81 |
| Méthionine | 4,67 | 14,45 |
| Isoleucine | 3,10 | 2,01 |
| Leucine | 9,29 | 3,58 |
| Tyrosine | 1,65 | 14,40 |
| Phénylalanine | 6,67 | 7,43 |
| Tryptophane | 1,41 | 2,13 |

et

i) la capacité de déclancher la formation d'anticorps spécifiques.

2. Procédé de concentration de la protéine tissulaire $PP_8$ suivant la revendication 1, caractérisé en ce que des extraits aqueux d'organes ou des solutions obtenues à partir de ceux-ci, sont soumis à au moins une des mesures suivantes:

1) précipitation par le sulfate d'ammonium dans le domaine de pH de 5 à 8 entre 40 et 70% de saturation;

2) précipitation par une base d'acridine soluble dans l'eau à un pH compris entre 7 et 9 et à une concentration de 0,4 à 0,8 g/100 cm³;

3) électrophorèse zonale préparative et obtention de la fraction d'alpha$_1$-globulines;

4) filtration sur gel pour recueillir des protéines dans le domaine de masses moléculaires de 30 000 à 70 000;

5) adsorption sur des échangeurs d'ions faiblement basiques et élution de la protéine tissulaire;

6) chauffage de la solution aqueuse en présence de sels d'acides gras aliphatiques pour précipiter des impuretés de la $PP_8$, et

7) enrichissement par immuno-adsorption, et en ce que la fraction enrichie en protéine tissulaire $PP_8$ est recueillie.

**Revendication pour l'Etat contractant: AT**
Procédé de concentration de la protéine tissulaire $PP_8$ ayant:

a) une teneur en protéines de $96 \pm 3\%$, une teneur en hydrates de carbone de $4,1 \pm 0,95\%$, parmi lesquels $3,15 \pm 0,5\%$ d'hexoses, $0,61 \pm 0,2\%$ d'hexosamine, $0,11 \pm 0,05\%$ de fucose, $0,23 \pm 0,20\%$ d'acide neuraminique;

b) un coefficient de sédimentation $S_{20,W}$ de $3,7 \pm 0,3$ S;

c) une masse moléculaire déterminée par ultracentrifugation de $45\,000 \pm 5000$;

d) une masse moléculaire déterminée dans un gel de polyacrylamide contenant du dodécylsulfate de sodium (SDS) de $55\,000 \pm 5000$;

e) un coefficient d'extinction $E^{1\%}_{1\,cm}$ (280 nm) de $8,1 \pm 1,0$;

f) une mobilité électrophorétique dans la région des $\alpha_1$-globulines;

g) un point isoélectrique de $4,7 \pm 0,3$;

h) la composition en aminoacides suivante:

|  | Moles % | Coefficient de variation (%) |
|---|---|---|
| Lysine | 7,08 | 3,21 |
| Histidine | 1,49 | 22,07 |
| Arginine | 3,56 | 3,86 |
| Acide aspartique | 10,32 | 6,79 |
| Thréonine | 6,08 | 0,96 |
| Sérine | 7,88 | 3,99 |
| Acide glutamique | 12,97 | 3,48 |
| Proline | 2,87 | 4,84 |
| Glycine | 6,64 | 2,19 |
| Alanine | 6,41 | 0,65 |
| Cystine/2 | 2,00 | 17,75 |
| Valine | 5,91 | 3,81 |
| Méthionine | 4,67 | 14,45 |
| Isoleucine | 3,10 | 2,01 |
| Leucine | 9,29 | 3,58 |
| Tyrosine | 1,65 | 14,40 |
| Phénylalanine | 6,67 | 7,43 |
| Tryptophane | 1,41 | 2,13 |

et

i) la capacité de déclancher la formation d'anticorps spécifiques, caractérisé en ce que des extraits aqueux d'organes ou des solutions obtenues à partir de ceux-ci, sont soumis à au moins und des mesures suivantes:

1) précipitation par le sulfate d'ammonium dans le domaine de pH de 5 à 8 entre 40 et 70% de saturation;

2) précipitation par une base d'acridine soluble dans l'eau à un pH compris entre 7 et 9 et à une concentration de 0,4 à 0,8 g/100 cm³;

3) électrophorèse zonale préparative et obtention de la fraction d'alpha$_1$-globulines;

4) filtration sur gel pour recueillir des protéines dans le domaine de masses moléculaires de 30 000 à 70 000;

5) adsorption sur des échangeurs d'ions faiblement basiques et élution de la protéine tissulaire;

6) chauffage de la solution aqueuse en présence de sels d'acides gras aliphatiques pour précipiter des impuretés de la $PP_8$, et

7) enrichissement par immuno-adsorption, et en ce que la fraction enrichie en protéine tissulaire $PP_8$ est recueillie.